# FASCICULE DE BREVET EUROPEEN

(11) **EP 0 436 697 B1**
(45) Date de publication et mention de la délivrance du brevet: **13.07.1994**
(21) Numéro de dépôt: 90911299.7
(22) Date de dépôt: 13.07.1990
(51) Int. Cl.: A61B 17/60, F16B 35/06, F16B 35/02, F16B 2/06, F16B 37/00, F16B 39/282

(54) **DISPOSITIF DE REDRESSEMENT, FIXATION, COMPRESSION, ELONGATION DU RACHIS**
VORRICHTUNG ZUM GERADERICHTEN, BEFESTIGEN, ZUSAMMENDRÜCKEN UND AUSDEHNEN DER WIRBELSÄULE
DEVICE FOR STRAIGHTENING, SECURING, COMPRESSING AND ELONGATING THE SPINAL COLUMN

(30) Priorité: 26.07.1989 FR 8910178
(43) Date de publication de la demande: 17.07.1991
(73) Titulaire: J.B.S. S.A., F-10000 Troyes (FR)
(72) Inventeur: MARNAY, Thierry, F-30870 Clarensac (FR)
(74) Mandataire: Gérardin, Robert Jean René
(86) Numéro de dépôt international: FR9000539
(87) Numéro de publication internationale: WO9101691

(56) Documents cités:
- EP-A- 0 128 058
- EP-A- 0 242 708
- WO-A-87/07134
- DE-A- 3 219 575
- DE-A- 3 639 810
- FR-A- 2 615 095
- FR-A- 2 624 720
- GB-A- 167 228
- GB-A- 2 173 104
- US-A- 3 752 203
- US-A- 4 411 259

## Description

L'invention concerne un dispositif pour le redressement et l'étaiement d'un rachis présentant une déviation anormale.

Le redressement et l'étaiement d'un rachis sont deux opérations nécessaires en cas de fracture de vertèbres, ou en cas de déviation de la colonne vertébrale résultant, par exemple, d'une scoliose ou d'une cyphose.

Les dispositifs connus de redressement et d'étaiement des rachis ont fait l'objet, depuis un certain nombre d'années de diverses réalisations. L'un des plus connus est celui universellement utilisé, dit de "HARRINGTON" dont nous ferons une brève description, dans ce qui suit, étant donné son éloignement relatif par rapport à l'invention.

Ce dispositif suscité comprend un système d'élongation et un système de compression, disposés respectivement sur la concavité et la convexité de la courbure du rachis ; le système d'élongation est constitué principalement d'une tige métallique cylindrique sur laquelle coulissent et peuvent être solidarisés de manière amovible deux éléments d'ancrage dits inférieur et supérieur ; l'élément inférieur est accroché sur la lame de la vertèbre extrême inférieure de la courbure et le crochet supérieur est disposé sous l'apophyse articulaire de la vertèbre extrême supérieure de la courbure ; l'une des extrémités de la tige est crantée de manière à permettre le réglage de l'écartement relatif des deux éléments d'ancrage, et à fixer la valeur de cet écartement par l'intermédiaire d'un clip de blocage ; le système de compression comporte une tige filetée sur laquelle coulissent des éléments d'ancrage, dont la position sur la tige peut être fixée par des écrous exerçant une pression sur les éléments et sur les vertèbres.

Une telle technique présente un certain nombre d'inconvénients tenant principalement au manque de précision du réglage de l'ouverture de la courbure due à la présence des crans, au fait que l'appui est localisé au niveau des deux seules vertèbres extrêmes de la courbure et que les crochets peuvent tourner autour de la tige métallique, aucune action de recentrage vers l'axe du tronc n'étant exercée, ni de dérotation des vertèbres du sommet, et au fait que la tige présente des risques de rupture.

La plupart de ces inconvénients ont été résolus par la création de dispositifs conduisant à la mise en place d'éléments d'ancrage sur un certain nombre de vertèbres de la courbure ; ces éléments sont constitués d'une partie réalisant l'ancrage, vis ou crochet, et d'un corps de fixation à la tige d'étaiement.

Il existe actuellement trois catégories de réalisation différentes des corps de fixation, à savoir les corps fermés, les corps ouverts latéralement et les corps ouverts longitudinalement.

Les premiers nécessitent le passage de la tige axialement dans le corps de fixation ; le blocage de la tige en rotation et en translation étant obtenu au moyen de petites vis introduites transversalement à la tige, dans un orifice du corps de fixation.

Or, le passage de ces tiges axialement dans le corps, après les avoir recourbées est une opération très contraignante et longue ; en outre, elle nécessite de procéder au hochement des corps de fixation des éléments d'ancrage dans lesquels la tige est déjà insérée ; ce qui provoque le dévissage des petites vis de pression assurant le blocage de ladite tige ; au surplus, ces petites vis mettent en évidence des parties saillantes, même lorsque celles-ci sont brisées à la fin de l'intervention, et pouvant être à l'origine de blessures et de lésions.

D'autres dispositifs remédient à certains de ces inconvénients en prévoyant des corps de fixation à ouverture latérale ; mais ils nécessitent toujours l'utilisation de vis de pression pour bloquer la tige.

Alors que d'autres dispositifs prévoient des corps de fixation à ouverture longitudinale tel que celui décrit dans le brevet suivant :
- Brevet européen 0 128 058, ayant pour objet un dispositif d'étaiement et de redressement du rachis dont le corps de fixation présente une ouverture longitudinale ; le blocage de la tige dans le corps étant obtenu par l'intermédiaire d'éléments complémentaires, dits bloqueurs, constitués de petits anneaux cylindro-coniques, surmontés d'un corps rectangulaire, le tout étant percé d'un trou taraudé destiné au vissage d'une vis de pression ; l'introduction de cet élément dans le corps de fixation, et le vissage de la vis de pression assurant la solidarisation axiale et radiale de la tige au corps de fixation.
   Ces dispositifs sont de conception onéreuse, compte-tenu du nombre important de pièces mises en oeuvre ; en outre, ils n'éliminent pas les parties sailiantes dues à la présence des vis ; ces éléments complémentaires devant en outre être placés sur la tige préalablement, nécessitent une orientation particulière des implants;
   Un autre dispositif combinant la possibilité d'introduction longitudinale des tiges et assurant un blocage de celles-ci par un nombre plus réduit de pièces, a été décrit dans la demande de brevet suivante :
- demande de brevet français 2 624 720 : dans laquelle le corps de fixation présente une ouverture longitudinale formant une rainure, fermée au moyen d'un capuchon vissé autour de la partie extérieure de la rainure ; le blocage définitif étant réalisé par l'intermédiaire d'une vis pointeau introduite dans un bossage du capuchon ; ce dispositif ne permet pas l'élimination des parties saillantes ; en outre la pression exercée sur la tige pour son blocage ne s'effectue que, d'une part au contact de deux bords opposés du capuchon, et, d'autre part, au niveau de la génératrice d'intersection entre la tige et le fond de la rainure, ce qui ne peut conduire à un blocage parfait ; bien que le blocage réalisé dans ce cas soit supérieur à celui réalisé par les dispositifs précédemment décrits.

La présente invention a pour but de remédier à ces inconvénients et se propose de résoudre le problème consistant à créer un dispositif d'étaiement et de redressement de conception simple et peu onéreuse, assurant une bonne fixation des implants sur les tiges à fonction d'étai, renforçant ainsi la solidité de l'ensemble ; sa mise en place étant rapide et précise et ne laissant subsister aucune partie saillante.

Le dispositif de redressement et d'étaiement d'un rachis, constitué d'implants vissés ou de crochets reliés par au moins deux tiges solidarisées entre elles par l'intermédiaire d'éléments de raccordement et de liaison ; lesdites tiges étant introduites dans des rainures perpendiculaires à la vis, prévues à cet effet dans le corps des implants ou des crochets puis bloquées dans le fond des rainures, une solidarisation entre des tiges étant obtenue par une traverse filetée, se caractérise en ce que le blocage des tiges dans le fond des rainures aménagées dans le corps des implants ou des crochets,est obtenu sous l'effet d'une déformation par rapprochement des côtés de la rainure par l'intermédiaire d'un système à vis cylindrique et à écrou à filetage conique, dans lequel, la solidarisation des tiges par rapport à la traverse filetée est obtenue par l'intermédiaire de quatre mors dont l'un est fixé à l'une des extrémités de la traverse filetée et dont la position des trois autres sur la traverse est réglable par l'intermédiaire d'un écrou maintenu dans une cage, dont la rotation s'obtient par l'intermédiaire d'un système roue et vis sans fin ; la fixation des vis des implants s'obtient par l'intermédiaire d'un filet triangulaire à 45°, dont l'un des côtés, situé en arrière par rapport à la pointe de la vis, forme un angle droit par rapport à la tige de la vis ; les éléments de raccordement longitudinaux de tronçons de tiges sont constitués d'une plaquette moletée dont les extrémités comportent chacune un orifice cylindrique de passage du corps de tête d'implants séparée ou non de sa vis sur laquelle se monte un écrou à filetage conique. Les éléments de liaison latéraux des tiges sont constitués d'une plaquette comportant un orifice de passage du corps de la tête d'un implant séparée ou non de sa tige, et une rainure de même largeur permettant elle aussi, le passage du corps de la tête d'un implant et d'écrous assurant le blocage en position de l'ensemble ; la tige filetée de rappel de deux crochets l'un vers l'autre est montée dans les orifices filetés de même pas, percés dans le corps desdits crochets.

Le système à vis cylindrique et à écrou conique, assurant le rapprochement des côtés du corps des implants ou des crochets, est constitué d'un filetage cylindrique mâle, réalisé autour du corps des implants ou des crochets sur une longueur un peu inférieure à la profondeur de la rainure et d'un alésage conique fileté de même pas, réalisé dans l'axe de l'écrou ; le diamètre de la base de l'alésage conique fileté de l'écrou correspond au diamètre du filetage cylindrique mâle réalisé autour du corps des implants ou des crochets; la hauteur de l'écrou correspond approximativement à la distance séparant l'entrée de la rainure de la tige, lorsque cette dernière est en place dans le fond de ladite rainure.

Suivant une forme particulière de réalisation de l'invention, la base de l'écrou comporte des dents disposées obliquement ; la section desdites dents a la forme d'un triangle rectangle dont la base est confondue avec celle de l'écrou et dont l'hypoténuse est dirigée dans le sens de rotation donné à l'écrou pour obtenir le vissage et le serrage de celui-ci.

Les dents disposées obliquement, peuvent être striées selon des cercles concentriques à l'axe de l'écrou.

Suivant une autre forme de réalisation de l'invention, les tiges servant d'étais sont filetées sur toute leur longueur, et le fond de la rainure, réalisée dans le corps des implants ou des crochets, comporte une empreinte demi-cylindrique dont le relief correspond à celui du filetage réalisé sur les tiges étais.

Les implants sont réalisés en titane pur, ou en alliage de titane implantable, par exemple en Ti Al6 V4.

La vis sans fin d'entraînement de la roue de manoeuvre de l'écrou, assurant le déplacement des mors mobiles, sur la traverse filetée, comporte une empreinte permettant sa manoeuvre par l'intermédiaire d'un tournevis ou d'une clé.

Le moyen de manoeuvre en rotation de la tige filetée de rappel des crochets deux à deux peut être constitué d'une empreinte permettant sa manoeuvre par l'intermédiaire d'un tournevis ou d'une clé.

Les avantages procurés par l'invention tiennent essentiellement en ceci que d'une part, le dispositif d'étaiement et de redressement du rachis ne laisse subsister aucune partie saillante, une fois mis en place, ceci étant obtenu grâce au blocage de la tige au moyen d'un écrou à tête arrondie et à alésage conique, ce qui élimine la nécessité d'utiliser des vis de pression ; d'autre part en ce que le blocage réalisé est optimum du fait que la pression exercée pour l'obtention du blocage est réalisée sur une surface d'appui importante de la tige ; ce blocage étant accru par la présence d'une denture sur la base de l'écrou coopérant avec des aspérités ménagées sur les tiges, ou d'une empreinte au fond de la rainure coopérant avec un filetage ménagé sur toute la longueur de la tige d'étaiement; le dispositif étant en outre de conception et d'utilisation simples, rapides et précises.

D'autres caractéristiques et avantages apparaîtront dans la description qui va suivre, de plusieurs formes de réalisation de l'invention, données à titre d'exemples non limitatifs, au regard des figures des planches annexées dans lesquelles :
- La figure 1 représente une vue en élévation longitudinale d'un implant à vis et une vue en élévation latérale d'un écrou, représentant, deux éléments constitutifs d'une forme particulière de réalisation de l'invention.
- La figure 2 est une représentation partielle de la vis, montrant son filet triangulaire.
- La figure 3 représente deux vues en élévation longitudinale de deux tiges à fonction d'étai, utilisées dans deux formes différentes de réalisation de l'invention.
- La figure 4 représente une vue en élévation latérale de l'écrou, utilisé dans la seconde forme de réalisation de l'invention.
- La figure 5 est une vue en plan de dessous, du même écrou, correspondant à une troisième forme de réalisation de l'invention.
- La figure 6 est une vue en coupe partielle suivant **A-A** de la figure précédente.
- La figure 7 représente une vue de dessus d'une vertèbre, le dispositif d'étaiement et de redressement de l'invention étant en place.
- La figure 8 représente une vue de dessus d'une forme de réalisation du dispositif de raccordement de deux tiges d'étaiement, disposées de part et d'autre d'un rachis, assurant leurs tension et écartement relatifs.
- La figure 9 est une vue de l'arrière du même dispositif présentant une coupe.
- La figure 10 est une vue dans un plan sagittal, du dispositif d'étaiement de l'invention mis en place.
- Les figures 11, 12, 13 et 14 représentent des vues en élévation longitudinale, de différentes formes de réalisation d'un crochet conforme à l'invention.
- Les figures 11a, 12a, 13a, et 14a en sont respectivement des vues de dessus, et la figure 13b est une vue de côté de la troisième forme de réalisation du crochet décrit.
- La figure 15 représente schématiquement une pince utilisée avec le dispositif de l'invention pour la manipulation des crochets.
- la figure 16 est une vue de côté partielle de la pince.
- la figure 17 représente, à travers trois vues, une agrafe utilisée avec le dispositif de l'invention.
- la figure 18 représente une vue partielle, dans un plan frontal, du dispositif de compression de deux vertèbres, conforme à l'invention.
- la figure 19 est une vue de côté du dispositif de compression de deux vertèbres.
- la figure 20 représente une vue de côté d'un dispositif de connexion longitudinale de tronçons de tiges.
- la figure 21 représente une vue de côté d'un dispositif de liaison latérale de tiges,
- la figure 22 représente une vue de dessus d'un dispositif de liaison latérale de tiges,

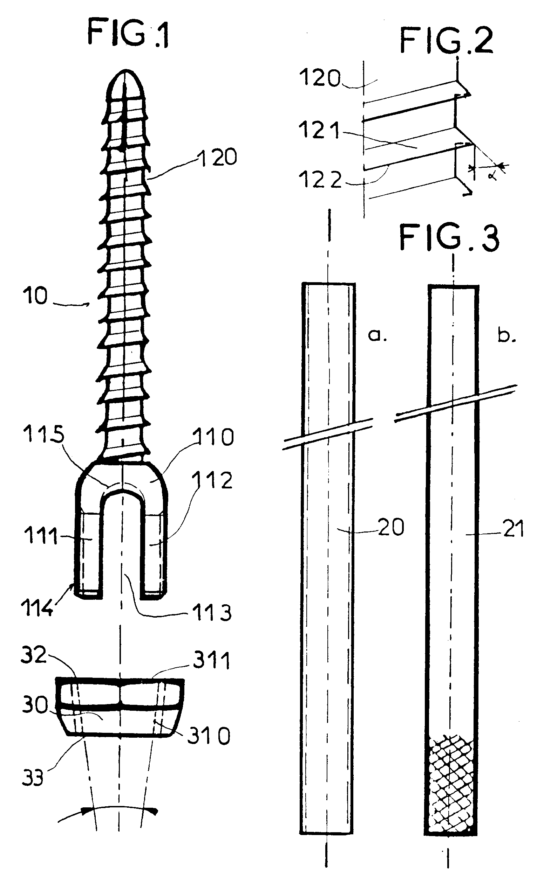

En se référant à la figure 1, on observe deux éléments constitutifs d'une forme particulière de réalisation de l'invention, à savoir un implant 10 et un écrou 30 ; dans cette forme de réalisation l'implant 10 prend la forme générale d'une vis dont la tête est formée d'un corps 110 présentant une rainure 113 ménagée dans le plan de la vis 120 de l'implant 10, débouchant à la partie postérieure du corps 110 de l'implant 10 ; et délimitée par deux côtés parallèles 111, 112 dudit implant 10, un filetage 114 est réalisé autour du corps 110, sur les trois quarts environ de la longueur de la rainure 113 et est destiné à recevoir le filetage de l'alésage 310 de l'écrou 30.

Lors de la mise en place du dispositif d'étaiement de l'invention, une tige à fonction d'étai est introduite, de chaque côté du rachis, successivement dans les rainures 113 de chaque corps 110 d'implants 10 ; ces derniers étant préalablement vissés dans les vertèbres situées entre les deux vertèbres extrêmes de la courbure d'un rachis, ou de part et d'autre d'une vertèbre fracturée, selon le cas ; puis la tige est bloquée au fond de la rainure 113 de chaque implant 10, par pression exercée sur celle-ci ; le fond de chaque rainure 113 est formé d'une partie semi-circulaire d'un diamètre correspondant à celui de la tige ; la pression exercée est réalisée lors du vissage de l'écrou 30 autour du corps 110 de l'implant 10, par le rapprochement des deux côtés 111, 112 de l'implant 10 délimitant la rainure 113, l'un par rapport à l'autre de manière à enserrer la tige et à la bloquer au fond de la rainure 113 ; ce rapprochement est réalisable grâce au choix d'un écrou 30 à alésage conique 310, le rapprochement obtenu des deux côtés 111, 112 étant proportionnel à la quantité de surface de l'alésage 310, vissée autour du corps 110 à de l'implant 10 ; le diamètre de la base 311 de l'alésage 310 de l'écrou 30 correspondant à celui du filetage cylindrique mâle 114 des implants 10.

Or, si ces moyens sus-décrits sont suffisants pour obtenir un blocage correct de la tige par rapport à l'implant 10, d'autres moyens ont été prévus pour renforcer ce blocage, de manière à augmenter la sécurité d'utilisation du dispositif de l'invention ; ces moyens sont la présence d'une empreinte demi-cylindrique 115 réalisée dans le fond de chaque rainure 113, dont le relief, en l'espèce un filetage en demi-lune est d'une forme lui permettant de coopérer avec le même relief réalisé sur toute la longueur des tiges d'étaiement ; ainsi la disposition de la tige au fond de la rainure 113 permettra d'obtenir la solidarisation axiale de la tige par rapport au corps 110 de l'implant 10, après le vissage de l'écrou 30 sur la totalité de sa hauteur ; la pression sera en outre exercée sur la tige par, d'une part, deux bords opposés de l'écrou 30, et, d'autre part, par les côtés de la rainure 113 sur au moins la moitié de la périphérie de la tige.

On pourra remarquer que la tête 33 de l'écrou 30 présente des bords arrondis et sa face extérieure opposée à la base 32, se situe lorsque l'écrou 30 est en place, dans le plan de l'extrémité des côtés 111, 112 de la rainure 113 ; en outre, les parties latérales de l'écrou 30 se retrouvent dans le prolongement des côtés non déformés du corps 110 de l'implant 10, de manière à ce que l'ensemble implant 10, écrou 30, ne présente plus aucune partie saillante.

Sur la figure 2, on remarque que la vis 120 reliée au corps de l'implant présente un filet triangulaire 121 formant un angle de 45°, la vis 120 peut dans certains cas d'utilisation être autotaraudeuse ; on notera que l'une des côtés 122, situé en arrière par rapport à la pointe de la vis 120, forme un angle droit par rapport à la tige de l'implant.

Sur la figure 3, on peut observer deux tiges 20,21 d'étaiement correspondant à celles utilisées dans les deux formes de réalisation différentes énoncées ci-dessus ; la première 20 (fig.3.a) est filetée sur toute sa longueur, de manière à coopérer avec le relief du fond de la rainure des implants ; la seconde 21 (fig.3b) présente des aspérités telles que celles pouvant résulter d'un moletage ou d'un guillochage, et pourra être utilisée dans la première forme de réalisation décrite, bien que l'utilisation, dans ce cas, d'une tige à surface lisse soit aussi possible ; mais cette seconde tige 21 est plus particulièrement destinée à être utilisée dans une troisième forme de réalisation du dispositif de l'invention que l'on va décrire dans ce que suit.

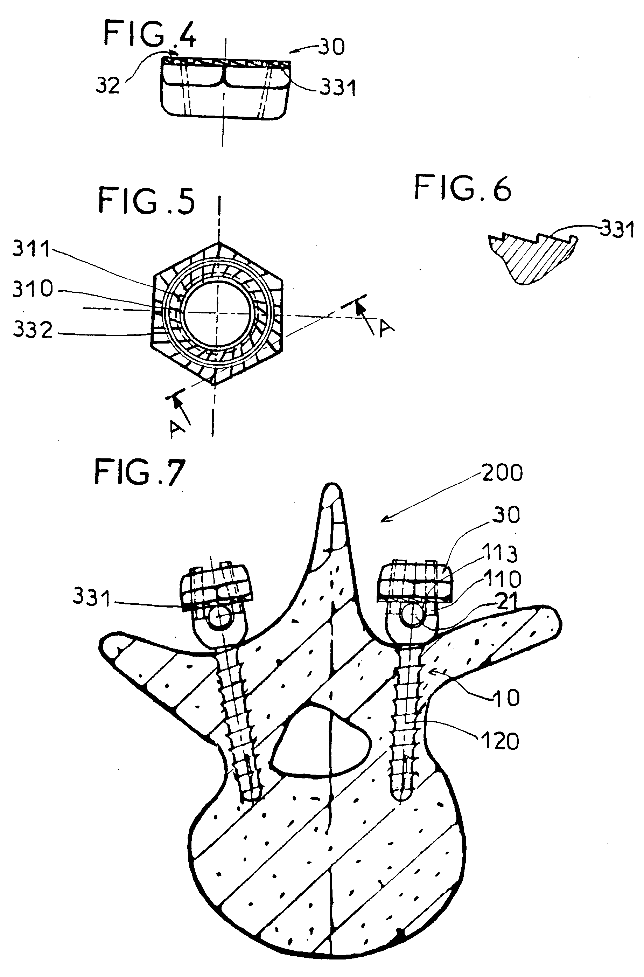

Sur les figures 4,5 et 6, l'écrou 30 est pourvu à sa base 32 d'une série de dents 331 destinées à coopérer avec les aspérités de la surface de la tige d'étaiement, de manière à ce qu'un glissement de l'écrou 30 par rapport à la surface de la tige soit obtenu lors du serrage final de celui-ci autour du corps de l'implant, et au contraire, à ce qu'un blocage de la tige soit obtenu par insertion des dents 331 dans les orifices des aspérités, lors d'un dévissage involontaire de l'écrou 30.

Ces dents 331 peuvent être disposées radialement, mais sont dans cette forme particulière de réalisation décrite, disposées de façon à être légèrement inclinées par rapport aux rayons de la base 311 de l'alésage 310 de l'écrou 30 ; cette particularité permet qu'un certain nombre de dents 331 soient toujours engagées dans les aspérités, lors du dévissage, ou en position de blocage ; ce que n'est pas obtenu lorsque les dents 331 sont disposées radialement ; afin d'obtenir un renforcement du blocage de l'écrou 30, on a également prévu une strie circulaire 332.

Sur la figure 7, on peut observer une vertèbre 200 dans laquelle sont vissés deux implants 10 à vis 120 ; la tige d'étaiement 21 ayant été mise en place dans les rainures 113 du corps 110 des implants 10 et solidarisée à ces derniers par l'intermédiaire d'un écrou 30 vissé autour du corps 110, dont la base est pourvue de dents 331, conforme à la dernière forme de réalisation décrite ; cette figure nous permet de considérer les surfaces d'appui par l'intermédiaire desquelles la pression de blocage est exercée sur la tige 21 ; lesquelles se trouvent d'une part au niveau de la base de l'écrou 30, d'autre part au niveau du fond de la rainure 113, sur un peu plus de la demi-périphérie de la tige 21.

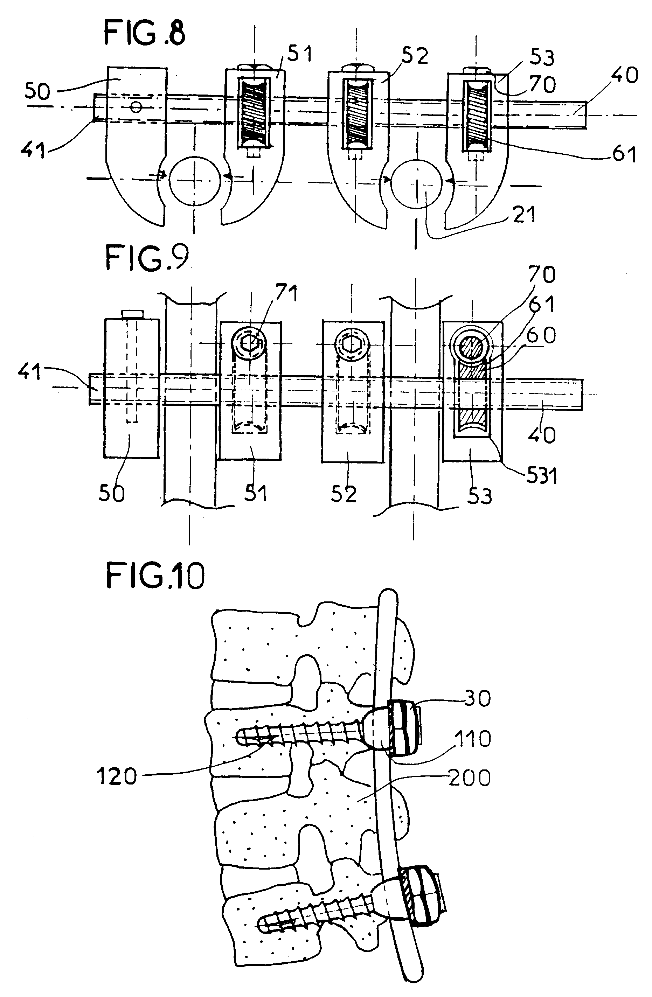

En se reportant aux figures 8 et 9, on peut observer une forme particulière de réalisation du dispositif de raccordement des deux tiges 20 et 21, mises en place de chaque côté d'un rachis.

Ce dispositif est constitué d'une traverse filetée 40 sur l'une des extrémités 41 de laquelle a été fixé un premier mors 50 ayant la forme générale d'un crochet, trois autres mors 51,52,53 pouvant se déplacer longitudinalement sur la traverse 40, par l'intermédiaire d'un écrou 60 maintenu dans une cage 531, solidaire du mors mobile 51,52,53 et entraîné en rotation par l'intermédiaire d'une roue à denture hélicoïdale 61 solidarisée autour de l'écrou 60 et entraînée elle-même en rotation par une vis sans fin 70 dont le filet est de même inclinaison que le denture de la roue 61 ; l'axe de ladite roue 61 étant orthogonal à l'axe de la vis 70 ; cette dernière étant manoeuvrable manuellement ou automatiquement grâce à la présence d'une empreinte hexagonale 71, par l'intermédiaire d'un outil approprié tel un tournevis ou une clé.

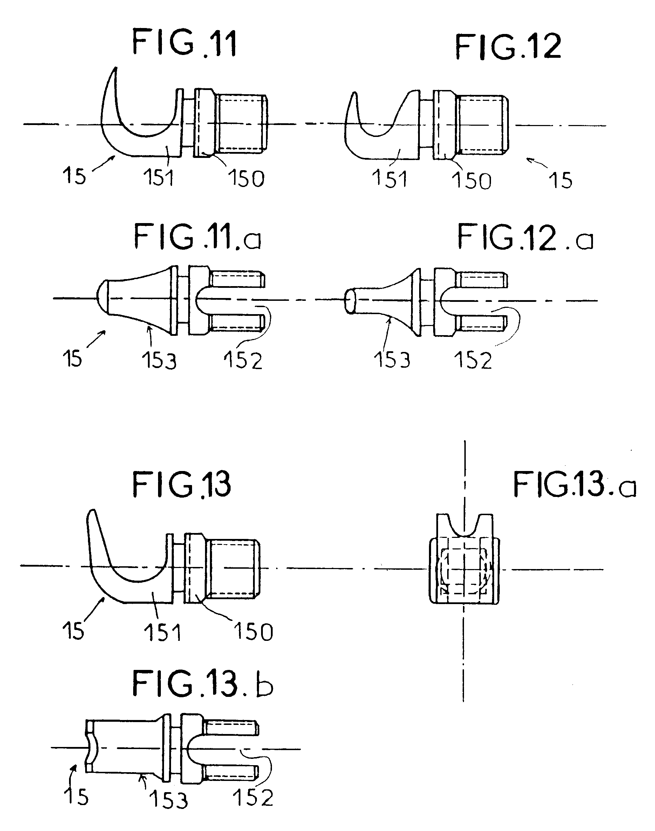

Sur la figure 10, on peut remarquer que le dispositif de l'invention, une fois mis en place, ne présente aucune partie saillante ; la vis 120 est vissée dans la vertèbre 200, les seules parties proéminentes étant l'écrou 30 et le corps de l'implant 10.

Sur les figures 11 à 14a, les éléments d'ancrage sont des crochets 15 constitués d'une part du crochet proprement dit 151 et d'un corps 150 présentant une rainure 152 destinée à recevoir une tige, de la même manière que pour les implants vissés décrits précédemment.

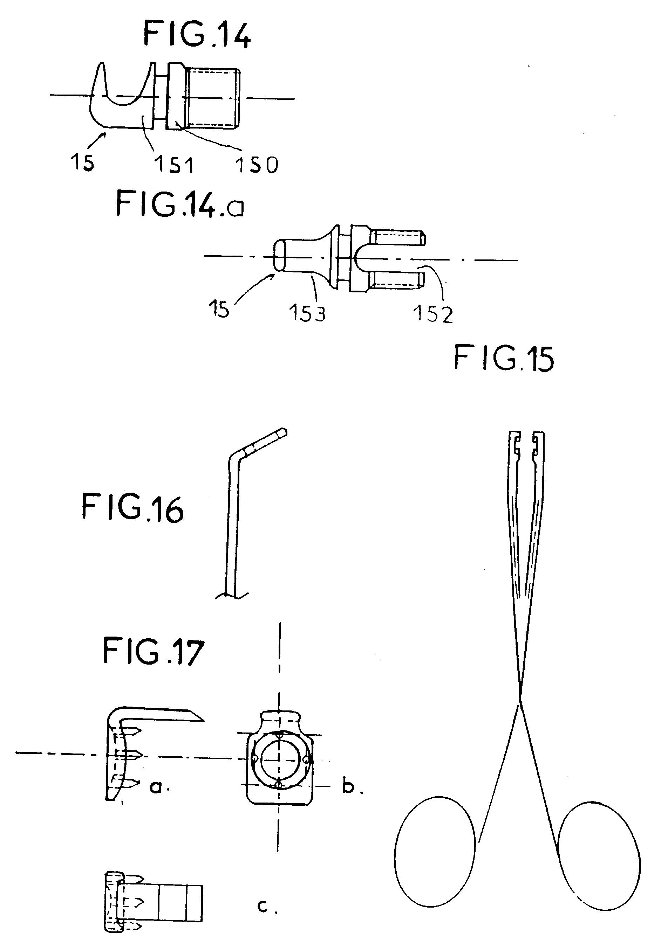

La forme de la partie intétieure des crochets 151 dépend de leur emplacement sur les vertèbres ; leur partie extérieure présente un décrochement 153 prévu pour leur manipulation au moyen d'une pince représentée sur les figures 15 et 16.

Sur la figure 17 (a,b,c), on peut observer une agrafe destinée à faciliter la mise en place et le maintien des implants dans les vertèbres .

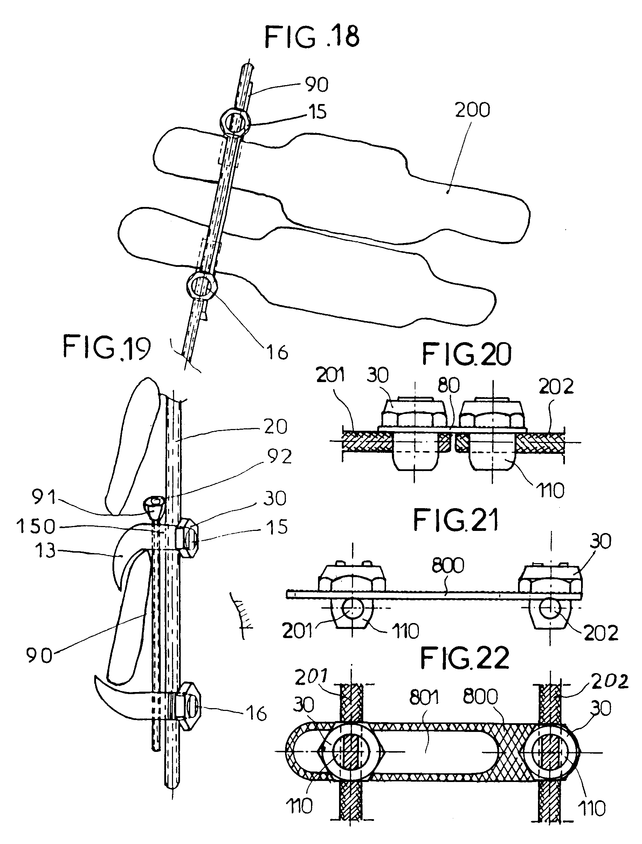

Sur les figures 18 et 19, le dispositif de compression, de deux vertèbres, l'une par rapport à l'autre est constitué, dans une forme particulière de réalisation, d'une tige filetée 90 et de deux crochets 15, 16 conformes à ceux de l'invention et comportant en outre, chacun, un orifice cylindrique fileté de même pas que la tige filetée 90 ; les deux crochets 15,16 étant de longueur différente, compte-tenu de la position inclinée des lames des vertèbres 200.

En position d'utilisation du dispositif, le corps 150 d'un des crochets 16 est fixé, de la manière sus-décrite, sur la tige d'étaiement 20, alors que le crochet est disposé sous la vertèbre inférieure et dirigé vers le haut ; l'autre crochet 15 est placé sur la vertèbre supérieure dirigée vers le bas, et peut coulisser le long de la tige 20 lorsqu'on visse la tige filetée 90 introduite dans les orifices respectifs des crochets 15,16 par l'intermédiaire d'une outil approprié (non représenté), coopérant avec une empreinte 92 ménagée sur la tête de vis 91, afin de rapprocher les deux crochets 15, 16 l'un par rapport à l'autre ; la fixation des crochets 15, 16 sur la tige 20 étant obtenue par l'intermédiaire d'un écrou 30, tel celui précédemment décrit.

De tels implants peuvent être utilisés pour le montage de plaques à arthrodèse lambo-sacrée sous réserve que l'extrémité supérieure de celles-ci comporte un tronçon de tiges de diamètre approprié permettant sa fixation aux implants fixés dans les vertèbres.

Sur la figure 20, le dispositif de raccordement longitudinal de tronçons de tiges 201 et 202, conforme à l'invention, est constitué d'une plaquette de raccordement 80 moletée sur ses deux faces, comportant deux orifices d'un diamètre correspondant à celui du corps 110 de la tête des implants 10 qui peuvent donc, sous réserve qu'elle soit séparée préalablement de sa vis 120, servir d'élément de raccordement des extrémités des tronçons de tiges 201 et 202, après que celles-ci aient été introduites dans la rainure de corps 110 constituant les éléments et que ceux-ci aient été introduits dans les orifices réalisés dans la plaquette. On comprend qu'il suffit ensuite de bloquer l'ensemble par l'intermédiaire des écrous 30 pour obtenir une solidarisation efficace des tiges 201 et 202.

Sur les figures 21 et 22, on remarque que la liaison latérale des tiges 201 et 202 peut être aisément obtenue par l'intermédiaire d'une plaquette moletée spéciale 800 s'inspirant de celle 80 utilisée pour le raccordement longitudinal, représentée à la figure 20 ; elle en diffère par l'allongement de ladite plaquette et le remplacement de l'un des orifices de passage des corps 110 des têtes d'implant, par une rainure 801 de largeur correspondants permettant l'adaptation de la liaison à l'écartement des tiges imposé par la position des implants 10 dans le corps duquel, elles sont fixées.

Les implants et écrous sont en titane ou alliage de titane implantable ; ce qui élimine les problèmes d'allergie, de nécrose (l'os se désagrège autour de lavis et ne tient plus bien), que l'on connaît avec l'utilisation d'implants en acier inoxydable, tels ceux utilisés dans les dispositifs connus.

Le dispositif est de mise en oeuvre aisée et rapide ; il est en effet possible de procéder au vissage des écrous autour des corps des implants au moyen d'un outil approprié, de façon automatique.

La précision de la mise en place est accrue.

Bien entendu, l'invention n'est pas limitée au mode de réalisation qui vient d'être décrit et représenté.
On pourra y apporter de nombreuses modifications de détail, sans sortir pour cela du cadre de l'invention.

## Revendications

1. Dispositif de redressement et d'étaiement d'un rachis constitué d'implants **(10)** vissés ou de crochets **(15)** reliés par au moins deux tiges **(20,21)** solidarisées entre elles par l'intermédiaire d'éléments de raccordement et de liaison ; lesdites tiges étant introduites longitudinalement dans des rainures **(113,152)** perpendiculaires à la vis, prévues à cet effet dans le corps des implants ou des crochets, puis bloquées dans le fond des rainures, une solidarisation entre des tiges étant obtenue par une traverse filetée **(40)**, caractérisé en ce qu'une déformation par rapprochement des côtés **(111, 112)** de la rainure, aménagée dans les corps (**110,150**) des implants **(10)** ou des crochets **(15)**, afin d'obtenir le blocage de la tige **(20 ou 21)** dans le fond de la rainure, est obtenu par l'intermédiaire d'un système à vis cylindrique **(114)** et à écrou à filetage conique **(30)**, en ce que la solidarisation de la tige **(20 ou 21)** par rapport à la traverse filetée **(40)** est obtenue par l'intermédiaire de quatre mors **(50,51,52,53)** dont l'un **(50)** est fixé à l'une des extrémités **(41)** de la traverse filetée **(40)**, et dont la position des trois autres **(51,52,53)** sur la traverse est réglable par l'intermédiaire d'un écrou **(60)** maintenu dans une cage **(531)**, dont la rotation s'obtient par l'intermédiaire d'un système roue **(61)** et vis sans fin **(70)**, en ce que la fixation des vis **(120)** des implants **(10)** s'obtient par l'intermédiaire d'un filet triangulaire **(121)** à 45°, dont l'un des côtés **(122)** situé en arrière par rapport à la pointe de la vis **(120)**, forme un angle droit par rapport à la tige de ladite vis **(120)**, en ce que des éléments de raccordement longitudinaux de tronçons de tiges **(201,202)** sont constitués d'une plaquette moletée **(80)** comportant deux orifices de passage du corps **(110)** de la tête d'implants **(10)** séparée ou non de sa vis **(120)**, sur laquelle se monte un écrou **(30)** qui assure le blocage de l'ensemble, en ce que des éléments de liaison latéraux des tiges **(21,22)** sont constitués d'une plaquette **(800)** comportant un orifice de passage du corps **(110)** de la tête d'un implant **(10)**, séparée ou non de sa tige, et une rainure de même largeur **(801)** permettant elle aussi, le passage du corps **(110)** de la tête d'un implant **(10)** et d'écrous **(30)** assurant le blocage en position de l'ensemble et en ce qu'une tige filetée **(90)** de rappel de deux crochets **(15, 16)**, l'un vers l'autre, est montée dans des orifices filetés de même pas, percés dans le corps **(150)** desdits crochets **(15, 16)**.

2. Dispositif selon la revendication 1, caractérisé en ce que le système à vis cylindrique **(114)** et à écrou conique **(30)** assurant le rapprochement des côtés du corps **(110, 150)** des implants **(10)** ou des crochets **(15)**, est constitué d'un filetage cylindrique mâle **(114)**, réalisé autour du corps **(110, 150)** des implants **(10)** ou des crochets **(15)**, à la profondeur de la rainure **(113 ou 152)**, et d'un alésage conique fileté **(310)** de même pas, réalisé dans l'axe de l'écrou **(30)**.

3. Dispositif selon la revendication 2, caractérisé en ce que le diamètre de la base **(311)** de l'alésage conique fileté **(310)** de l'écrou **(30)** correspond au diamètre du filetage cylindrique mâle **(114)** réalisé autour du corps **(110, 150)** des implants **(10)** ou des crochets **(15)**.

4. Dispositif selon les revendications 1 ou 2, caractérisé en ce que la hauteur de l'écrou **(30)** correspond approximativement à la distance séparant l'entrée de la rainure **(113** ou **152)** de la tige **(20** ou **21)**, lorsque cette dernière est en place dans le fond de ladite rainure.

5. Dispositif selon la revendication 1, caractérisé en ce que la tête **(33)** de l'écrou **(30)** est arrondie alors que sa base **(32)** comporte des dents **(331)** disposées obliquement.

6. Dispositif selon la revendication 5, caractérisé en ce que la section des dents **(331)** de l'écrou **(30)** a la forme d'un triangle rectangle dont la base est confondue avec celle de l'écrou **(30)**.

7. Dispositif selon la revendication 6, caractérisé en ce que l'hypoténuse du triangle rectangle est dirigée dans le sens de rotation donné à l'écrou **(30)** pour obtenir le vissage et le serrage de celui-ci.

8. Dispositif selon la revendication 5, caractérisé en ce que les dents **(331)**, disposées obliquement, sont striées selon des cercles **(332)** concentriques à l'axe de l'écrou **(30)**.

9. Dispositif selon la revendication 1, caractérisé en ce que la tige **(20 ou 21)** servant d'étais est filetée sur toute sa longueur.

10. Dispositif selon les revendications 1 et 2 caractérisé, en ce que le fond de la rainure **(113 ou 152)** réalisée dans le corps **(110)** des implants **(10)** ou des crochets **(15)**, comporte une empreinte demi-cylindrique **(115)** dont le relief correspond à celui du filetage réalisé sur la tige étai **(20)**.

11. Dispositif selon la revendication 1, caractérisé en ce que les implants **(10)** sont réalisés en titane pur Ti ou en alliage de titane implantable.

12. Dispositif selon la revendication 11, caractérisé en ce que les implants sont réalisés en Ti Al6 V4.

13. Dispositif selon la revendication 1, caractérisé en ce que la vis sans fin **(70)** d'entraînement de la roue **(61)** de manoeuvre de l'écrou **(60)**, assurant le déplacement des mors mobiles **(51,52,53)** sur la traverse filetée **(40)**, comporte un empreinte **(71)** permettant sa manoeuvre par l'intermédiaire d'un tournevis ou d'une clé.

14. Dispositif selon la revendication 1, caractérisé en ce que le moyen de manoeuvre en rotation de la tige filetée de rappei **(90)** des crochets **(15, 16)**, deux à deux, est constitué d'une empreinte **(92)** permettant sa manoeuvre par l'intermédiaire d'un tournevis ou d'une clé.

## Claims

1. Device for straightening and supporting a rachis, consisting of screwed-in implants (10) or hooks (15) connected by at least two rods (20, 21) fixed to each other by means of joining and connecting members; the said rods being inserted longitudinally into recesses (113, 152) perpendicular to the screw and provided for this purpose in the body of the implants or hooks, and then locked in the bottom of the recesses, the fixing of the rods to each other being obtained by means of a threaded cross piece (40), characterised in that a deformation caused by bringing closer together the sides (111, 112) of the recess produced in the body (110, 150) of the implants (10) or of the hooks (15), in order to obtain the locking of the rod (20 or 21) in the bottom of the recess, is obtained by means of a system consisting of a cylindrical screw (114) and a nut with a tapered screw thread (30), in that the fixing of the rod (20 or 21) with respect to the threaded cross piece (40) is obtained by means of four jaws (50, 51, 52, 53), one of which (50) is fixed to one end (41) of the threaded cross piece (40), the position of the three others (51, 52, 53) on the cross piece being adjustable by means of a nut (60) held in a housing (531), the rotation of which is obtained by means of a system consisting of a gear (61) and worm (70), in that the fixing of the screws (120) of the implants (10) is obtained by means of an angular thread (121) at an angle of 45°, one of the edges of which (122), situated behind with respect to the shank of the screw (120), forms a right angle with respect to the rod of the said screw (120), in that components for the longitudinal connection of sections of rods (201, 202) consist of a knurled plate (80) having two orifices for the body (110) of the head of the implants (10) to pass through, whether or not the head is separate from its screw (120), on which is mounted a nut (30) which provides the locking of the assembly, in that components for the lateral connection of the rods (21, 22) consist of a plate (800) having an orifice for the body (110) of the head of an implant (10) to pass through, whether or not the head is separate from its rod, and a recess of the same width (801) also enabling the body (110) of the head of an implant (10) and nuts (30) to pass through, locking the assembly in position, and in that a threaded rod (90) pulling two hooks (15, 16) towards each other is mounted in threaded orifices having the same thread pitch, formed in the body (150) of the said hooks (15, 16).

2. Device according to claim 1, characterised in that the system consisting of a cylindrical screw (114) and tapered nut (30) bringing closer together the sides of the body (110, 150) of the implants (10) or hooks (15) consists of a male screw thread (114) formed round the body (110, 150) of the implants (10) or hooks (15) to the depth of the recess (113 or 152), and a tapered threaded bore (310) with the same thread pitch, formed in the axis of the nut (30).

3. Device according to Claim 2, characterised in that the diameter of the base (311) of the conical threaded bore (310) of the nut (30) corresponds to the diameter of the male screw thread (114) formed around the body (110, 150) of the implants (10) or hooks (15).

4. Device according to Claim 1 or 2, characterised in that the height of the nut (30) corresponds approximately to the distance separating the mouth of the recess (113 or 152) from the rod (20 or 21), when the latter is in position in the bottom of the said recess.

5. Device according to Claim 1, characterised in that the crown (33) of the nut (30) is rounded whilst its base (32) has teeth (331) disposed obliquely.

6. Device according to claim 5, characterised in that the cross section of the teeth (331) of the nut (30) has the shape of a right-angled triangle, the base of which is identical with that of the nut (30).

7. Device according to Claim 6, characterised in that the hypotenuse of the right-angled triangle is directed in the direction of rotation given to the nut (30) in order to obtain the screwing and tightening of the latter.

8. Device according to Claim 5, characterised in that the teeth (331), disposed obliquely, are laid out in circles (332) concentric with the axis of the nut (30).

9. Device according to Claim 1, characterised in that the rod (20 or 21) acting as a support is threaded over its entire length.

10. Device according to Claims 1 and 2, characterised in that the bottom of the recess (113 or 152) formed in the body (110) of the implants (10) or of the hooks (15) has a semi-cylindrical indentation (115), the profile of which corresponds to that of the thread formed in the supporting rod (20).

11. Device according to Claim 1, characterised in that the implants (10) are made of pure titanium Ti or an implantable titanium alloy.

12. Device according to Claim 11, characterised in that the implants are made of TI AI6 V4 titanium.

13. Device according to Claim 1, characterised in that the worm (70) for driving the gear (61) for manoeuvring the nut (60), ensuring the movement of the movable jaws (51, 52, 53) on the threaded cross piece (40), has an indentation (71) enabling it to be manoeuvred by means of a screwdriver or key.

14. Device according to Claim 1, characterised in that the means for manoeuvring in rotation the threaded rod (90) pulling together the hooks (15, 16), in pairs, consists of an indentation (92) enabling it to be manoeuvred by means of a screwdriver or key.

## Patentansprüche

1. Vorrichtung zum Geraderichten und Stützen einer Wirbelsäule, bestehend aus geschraubten Implantaten (10) oder Haken (15), die durch mindestens zwei Stangen (20, 21) verbunden sind, die über Anschluß- und Verbindungselemente untereinander zusammengehalten werden, wobei diese Stangen längs in Nuten (113, 152) eingeführt werden, die lotrecht zur Schraube verlaufen und zu diesem Zweck im Körper der Implantate oder Haken vorgesehen sind, und dann blockiert am Grund der Nuten blockiert sind, wobei ein Zusammenhalt zwischen den Stangen über eine Gewindetraverse (40) erreicht wird, **dadurch gekennzeichnet, daß** eine Verformung durch Annäherung der Seiten (111, 112) der in den Körpern (110, 150) der Implantate (10) oder Haken (15) vorgesehenen Nut zum Erreichen der Klemmung der Stange (20 bzw. 21) am Gund der Nut über ein System mit Zylinderschraube (114) und Mutter mit konischem Gewinde (30) erreicht wird, daß der Zusammenhalt der Stange (20 bzw. 21) in Bezug auf die Gewindetraverse (40) über vier Spannbacken (50, 51, 52, 53) erreicht wird, von denen eine (50) an einem Ende (41) der Gewindetraverse (40) befestigt ist und wobei die Lage der übrigen drei (51, 52, 53) auf der Traverse über eine Mutter (60) regulierbar ist, die in einem Einsatz (531) gehalten wird, dessen Drehung über ein System mit Rad (61) und Schnecke (70) erreicht wird, daß die Befestigung der Schrauben (120) der Implantate (10) über ein 45°-Spitzgewinde (121) erzielt wird, dessen eine Seite (122), hinten liegend in Bezug auf die Spitze der Schraube (120), einen rechten Winkel zur Stange dieser Schraube (120) bildet, daß Längsverbindungselemente von Stangenabschnitten (201, 202) aus einem Rändelplättchen (80) bestehen, das zwei Öffnungen zum Durchführen des Körpers (110) des Implantatkopfes (10) besitzt, der von seiner Schraube (120) getrennt ist oder nicht, auf die eine Mutter (30) montiert wird, die die Klemmung des Ganzen gewährleistet, daß seitliche Verbindungselemente der Stangen (21, 22) aus einem Plättchen (800) bestehen, das eine Durchgangsöffnung für den Körper (110) des Kopfes eines Implantats (10) besitzt, von seiner Stange getrennt oder nicht, und eine Nut derselben Breite (801), die ebenfalls das Durchführen des Körpers (110) des Kopfes eines Implantats (10) und von Muttern (30) erlaubt, die die Klemmung des Ganzen in der richtigen Lage gewährleisten, und daß eine Rückstellgewindestange (90) für zwei Haken (15, 16) gegeneinander in Gewindelöchern mit dem gleichen Gewinde montiert ist, die in den Körper (150) dieser Haken (15, 16) gebohrt sind.

2. Vorrichtung nach Anspruch 1, **dadurch gekennzeichnet, daß** das System mit Zylinderschraube (114) und konischer Mutter (30) zur Annäherung der Seiten des Körpers (110, 150) der Implantate (10) oder Haken (15) aus einem zylindrischen Außengewinde (114) rund um den Körper (110, 150) der Implantate (10) oder Haken (15) bis auf die Tiefe der Nut (113 bzw. 152) und einer konischen Gewindebohrung (310) derselben Steigung in der Achse der Mutter (30) besteht.

3. Vorrichtung nach Anspruch 2, **dadurch gekennzeichnet daß** der Durchmesser der Basis (311) der konischen Gewindebohrung (310) der Mutter (30) dem Durchmesser des zylindrischen Außengewindes (114) rund um den Körper (110, 150) der Implantate (10) oder Haken (15) entspricht.

4. Vorrichtung nach den Ansprüchen 1 oder 2, **dadurch gekennzeichnet, daß** die Höhe der Mutter (30) ungefähr dem Abstand zwischen dem Eingang der Nut (113 oder 152) und der Stange (20 oder 21) entspricht, wenn sich die Stange am Grund dieser Nut befindet.

5. Vorrichtung nach Anspruch 1, **dadurch gekennzeichnet, daß** der Kopf (33) der Mutter (30) abgerundet ist, während ihre Basis (32) schräg angeordnete Zähne (331) hat.

6. Vorrichtung nach Anspruch 5, **dadurch gekennzeichnet, daß** der Querschnitt der Zähne (331) der Mutter (30) die Form eines rechtwinkligen Dreiecks hat, dessen Basis in diejenige der Mutter (30) übergeht.

7. Vorrichtung nach Anspruch 6, **dadurch gekennzeichnet, daß** die Hypotenuse des rechtwinkligen Dreiecks zur Drehrichtung der Mutter (30) geneigt ist, damit diese eingeschraubt und angezogen werden kann.

8. Vorrichtung nach Anspruch 5, **dadurch gekennzeichnet, daß** die schräg angeordneten Zähne (331) mit konzentrischen Kreisen (332) zur Achse der Mutter (30) gerieft sind.

9. Vorrichtung nach Anspruch 1, **dadurch gekennzeichnet, daß** die als Stütze dienende Stange (20 oder 21) auf ihrer ganzen Länge mit einem Gewinde versehen ist.

10. Vorrichtung nach den Ansprüchen 1 und 2, **dadurch gekennzeichnet, daß** der Grund der Nut (113 oder 152) im Körper (110) der Implantate (10) oder Haken (15) eine halbzylindrische Vertiefung (115) besitzt, deren Relief dem des Gewindes auf der Stützstange (20) entspricht.

11. Vorrichtung nach Anspruch 1, **dadurch gekennzeichnet, daß** die Implantate (10) aus reinem Titan Ti oder aus einer implantierbaren Titanlegierung hergestellt sind.

12. Vorrichtung nach Anspruch 11, **dadurch gekennzeichnet, daß** die Implantate aus Ti Al6 V4 hergestellt sind.

13. Vorrichtung nach Anspruch 1, **dadurch gekennzeichnet, daß** die Antriebsschnecke (70) des Rades (61) der Mutter (60) zum Verschieben der beweglichen Backen (51, 52, 53) auf der Gewindetraverse (40) eine Vertiefung (71) besitzt, um mit einem Schraubenzieher oder Schlüssel betätigt zu werden.

14. Vorrichtung nach Anspruch 1, **dadurch gekennzeichnet, daß** das Mittel zum Drehen der Rückstellgewindestange (90) der beiden Haken (15, 16) aus einer Vertiefung (92) besteht, um mit einem Schraubenzieher oder Schlüssel betätigt zu werden.
